# EUROPÄISCHE PATENTSCHRIFT

(11) **EP 0 889 019 B1**
(45) Veröffentlichungstag und Bekanntmachung des Hinweises auf die Patenterteilung: **31.10.2001**
(21) Anmeldenummer: 98111054.7
(22) Anmeldetag: 17.06.1998
(51) Int. Cl.: C07C 45/00, C07C 49/417

(54) **Verfahren zur Herstellung von Cyclohexanonen durch Hydrierung der entsprechenden Phenole in Gegenwart von Alkanen als Lösungsmittel**
Process for preparing cyclohexanones by hydrogenation of the corresponding phenols in presence of alkanes as solvent
Procédé pour la préparation de cyclohéxanes par hydrogénation des phénols correspondants en présence d'alkanes comme solvant

(30) Priorität: 30.06.1997 DE 19727710
(43) Veröffentlichungstag der Anmeldung: 07.01.1999
(73) Patentinhaber: BAYER AG, 51368 Leverkusen (DE)
(72) Erfinder: Kiel, Wolfgang, Dr., 51519 Odenthal (DE)

(56) Entgegenhaltungen:
- EP-A- 0 731 075
- US-A- 4 304 943

## Beschreibung

Die vorliegende Erfindung betrifft ein vorteilhaftes Verfahren zur Hydrierung von Phenolen zu den entsprechenden Cyclohexanonen unter Verwendung von Palladium-auf-Kohle-Katalysatoren.

Verfahren zur Hydrierung von Phenolen zu Cyclohexanonen an Palladium-auf-Kohle-Katalysatoren sind grundsätzlich bekannt (siehe z.B. DE-A 2 909 780, DE-A 2 530 759 und US-A 2 829 166).

In der Regel wird zum Erreichen einer guten Selektivität ein Wasserstoffdruck von weniger als 20 bar und eine Reaktionstemperatur im Bereich von 120 bis 250°C angestrebt und dem Hydriergemisch eine alkalisch reagierende Verbindung zugefügt. Der Katalysator kann gegebenenfalls mit Alkalimetallionen wie Na- oder K-Ionen behandelt werden (siehe z.B. DE-AS 1 144 267). Der Einsatz von Phenolaten und Bi-carbonaten ist in der FR-A 2 372 136 beschrieben.

Nach der DE-A 2 909 780 wird zur Hydrierung von p-tert.-Amylphenol Natriumcarbonat, Borax und/oder Natriumacetat eingesetzt. Gleichzeitig kommt hier zum Ausdruck, daß der Einsatz von Palladium-Katalysatoren mit Metalloberflächen von 15 m²/g und mehr erfolgversprechend ist. Bevorzugt werden Palladium-auf-Kohle-Katalysatoren eingesetzt, die 5 Gew.-% Palladium enthalten, bezogen auf einen Träger, der eine Oberfläche von 800 m²/g aufweist.

Ein Nachteil dieser bekannten Verfahren liegt darin, daß für die Umsetzung von Phenolen mit hohen Schmelzpunkten aus technischer und/oder toxikologischer Sicht nur schwierig handhabbare Lösungsmittel bekannt sind (s. z.B. EP-A 731 075). Erwünscht wären Lösungsmittel, die sicherheitstechnisch und toxikologisch sicher zu handhaben und physikalisch problemlos mit geringem Aufwand abtrennbar sind.

Außerdem werden, insbesondere wenn bei der Hydriertemperatur flüssige Phenole und keine Lösungsmittel eingesetzt werden, häufig lange Reaktionszeiten benötigt.

Es wurde nun ein Verfahren zur Herstellung von Cyclohexanonen der Formel (I) gefunden, 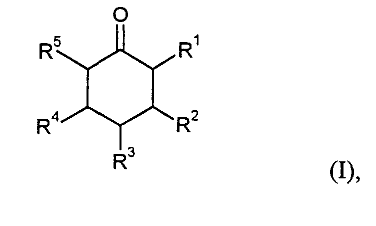 in der
- R¹, R², R³, R⁴ und R⁵: unabhängig voneinander jeweils für Wasserstoff, Hydroxy, C₁-C₁₀-Alkyl, C₃-C₆-Cycloalkyl, C₆-C₁₀-Aryl, C₆-C₁₀-Aryl-CH₂-, C₆-C₁₀-Aryl-O-, C₁-C₁₀-Alkoxy, C₃-C₈-Cycloalkoxy, C₁-C₄-Alkylamino, Di-C₁-C₄-alkylamino, C₁-C₄-Acylamino, COOR⁶ mit R⁶ = Wasserstoff oder C₁-C₄-Alkyl oder R⁷-CH₂- mit R⁷ = Hydroxy, C₁-C₄-Alkoxy, C₁-C₄-Alkylamino oder Di-C₁-C₄-alkylamino bedeuten,
durch Hydrierung von Phenolen der Formel (II) 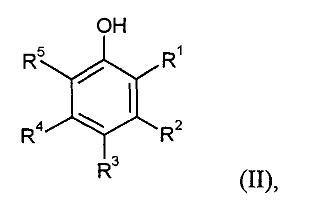 in der R¹ bis R⁵ die bei Formel (I) angegebene Bedeutung haben,
in Gegenwart eines Palladium-auf-Kohle-Katalysators bei 100 bis 250°C und 1 bis 20 bar Wasserstoffdruck, das dadurch gekennzeichnet ist, daß man in Gegenwart von geradkettigen, verzweigten oder cyclischen Alkanen mit einem Siedepunkt bei Normaldruck von über 70°C als Lösungsmittel arbeitet.

In den Formeln (I) und (II) stehen R¹, R², R³, R⁴ und R⁵ unabhängig voneinander vorzugsweise für Wasserstoff, Hydroxy, C₁-C₆-Alkyl, C₁-C₆-Alkoxy oder C₁-C₄-Acylamino. Außerdem ist es bevorzugt, daß 1 bis 4 der Reste R¹ bis R⁵ von Wasserstoff verschieden sind.

Beispiele für C₁-C₆-Alkyl sind Methyl, Ethyl, i-Propyl, t-Butyl und Pentyl. Beispiele für C₁-C₆-Alkoxy sind Methoxy, Ethoxy und i-Propoxy. Ein Beispiel für C₁-C₄-Acylamino ist Acetylamino.

Besonders bevorzugt stehen R¹, R², R⁴ und R⁵ für Wasserstoff und R³ für Hydroxy, C₁-C₆-Alkyl, C₁-C₆-Alkoxy oder C₁-C₄-Acylamino.

Die Palladium-auf-Kohle-Katalysatoren können z.B. 1 bis 15 Gew.-% Palladium auf einer beliebigen Kohle enthalten. Vorzugsweise enthalten solche Katalysatoren 2 bis 12 Gew.-% Palladium. Derartige Katalysatoren sind im Handel erhältlich. Häufig werden sie in wasserfeuchter Form angeboten und können auch in dieser wasserfeuchten Form eingesetzt werden. Bezogen auf das Phenol der Formel (II) kann man beispielsweise 0,001 bis 1 Gew.-% Katalysator (berechnet als Pd-Metall) einsetzen. Bevorzugt beträgt diese Menge 0,05 bis 0,2 Gew.-%.

Die Reaktionstemperatur liegt vorzugsweise bei 150 bis 200°C und der Wasserstoffdruck vorzugsweise bei 3 bis 8 bar.

Es ist ein wesentliches Merkmal der vorliegenden Erfindung, daß man als Lösungsmittel geradkettige, verzweigte oder cyclische Alkane einsetzt, die bei Normaldruck einen Siedepunkt von über 70°C aufweisen. In Frage kommen beispielsweise geradkettige und verzweigte C₇-C₁₈-Alkane, unsubstituierte C₆-C₁₀-Cycloalkane und mit geradkettigen oder verzweigten C₁-C₁₀-Alkylgruppen substituierte C₆-C₁₀-Cycloalkane. Bevorzugte Lösungsmittel sind Isooctan, Cyclohexan und Methylcyclohexan. Besonders bevorzugt ist Methylcyclohexan. Es können auch Gemische verschiedener Lösungsmittel verwendet werden.

Bezogen auf 100 g des eingesetzten Phenols der Formel (II) kann man beispielsweise 10 bis 1000 ml der erfindungsgemäß zu verwendenden Lösungsmittel einsetzen. Vorzugsweise beträgt diese Menge 50 bis 300 ml.

In einer besonderen Ausführungsform des erfindungsgemäßen Verfahrens setzt man den Katalysator nicht als solchen oder in wasserfeuchter Form ein, sondern vermischt ihn vorher mit einer Basenkomponente und 20 bis 200 Gew.-% Wasser (bezogen auf die Basenkomponente). Als Basenkomponente kommen insbesondere in wäßrigem Milieu basisch reagierende Alkalisalze infrage. Bevorzugt sind Borax (Na₂B₄O₇x10H₂O), Natriumacetat und Dinatriumhydrogenphosphat. Bezogen auf den Palladium-auf-Kohle-Katalysator kann man beispielsweise 5 bis 50 Gew.-% Basenkomponente einsetzen. Vorzugsweise beträgt diese Menge 10 bis 20 Gew.-%.

Die Wassermenge bezogen auf die Basenkomponente beträgt vorzugsweise 50 bis 150 Gew.-%. Bei der einzusetzenden Wassermenge wird in der Basenkomponente eventuell vorhandenes Kristallwasser nicht berücksichtigt. Ebenso wird das Wasser nicht berücksichtigt, das evtl. für die Befeuchtung des Katalysators zu seiner besseren Handhabung verwendet wurde. Es ist vorteilhaft, zunächst die Basenkomponente mit Wasser zu vermischen, wobei es unerheblich ist, ob sich die Basenkomponente vollständig löst oder nicht, und dann das Basenkomponente/Wasser-Gemisch mit dem trockenen oder wasserfeuchten Palladium-auf-Kohle-Katalysator zu vermischen.

Prinzipiell kann man zwar Phenole der Formel (II), die einen Schmelzpunkt unterhalb der gewünschten Hydriertemperatur aufweisen, in der Schmelze und ohne Lösungsmittelzusatz hydrieren. Es hat sich jedoch gezeigt, daß auch in diesen Fällen der Zusatz von erfindungsgemäß anzuwendenden Lösungsmitteln vorteilhaft ist, weil dadurch die Hydriergeschwindigkeit stark erhöht wird.

Das erfindungsgemäße Verfahren kann beispielsweise in Rühr- oder Schlaufenreaktoren durchgeführt werden.

Das erfindungsgemäße Verfahren hat die Vorteile, daß man mit ihm unter Verwendung von sicherheitstechnisch gut zu handhabenden, wenig toxischen, physikalisch problemlosen und gut abtrennbaren Lösungsmitteln Cyclohexanone der Formel (I) in kurzen Hydrierzeiten und hohen Ausbeuten herstellen kann. Häufig bildet das Lösungsmittel nach der Reaktion eine separate, praktisch katalysator- und produktfreie Phase, die einfach abgetrennt und im nächsten Ansatz wieder verwendet werden kann. So kann man beispielsweise bei der Hydrierung von Hydrochinon zu p-Hydroxycyclohexanon in Gegenwart von Methylcyclohexan als Lösungsmittel verfahren.

### Beispiele

### Beispiel 1

0,5 g Borax und 0,5 g Wasser wurden vermischt und dann 6 g eines wasserfeuchten Katalysators (Wassergehalt 50 %) zugemischt, der 5 Gew.-% Palladium auf Kohlen enthielt. Das so erhaltene Gemisch wurde zu einer Lösung von 100 g p-Methoxyphenol in 150 g Methylcyclohexan gegeben und dieses Reaktionsgemisch in einem Rührreaktor bei 150 bis 180°C und 3 bis 8 bar Wasserstoffdruck hydriert bis kein Wasserstoff mehr aufgenommen wurde. Nach einer Hydrierzeit von 72 Minuten hatten sich 96,2 Gew.-% des p-Methoxyphenols umgesetzt, und p-Methoxycyclohexanon hatte sich in einer Ausbeute von 83 Gew.-% gebildet.

### Beispiel 2 (zum Vergleich)

Es wurde verfahren wie in Beispiel 1, jedoch wurde das p-Methoxyphenol in geschmolzener Form ohne Zusatz von Methylcyclohexan eingesetzt. Nach einer Hydrierzeit von 380 Minuten hatten sich 62,1 Gew.-% des p-Methoxyphenols umgesetzt, und p-Methoxycyclohexanon hatte sich in einer Ausbeute von 46,5 Gew.-% gebildet.

### Beispiel 3 bis 5

Es wurde verfahren wie in Beispiel 1, jedoch wurden andere Phenole in entsprechender Menge eingesetzt. Einzelheiten und Ergebnisse sind aus der Tabelle 1 ersichtlich.

**Tabelle 1**

| Beispiel Nr. | eingesetztes Phenol | Hydrierzeit (min) | erhaltenes Cyclohexanon (Gehalt im Reaktionsgemisch) |
|---|---|---|---|
| 3 | p-tert.-Butylphenol | 53 | p-tert.-Butylcyclohexanon (91,7 %) |
| 4 | Hydrochinon | 165 | p-Hydroxycyclohexanon (88,0%) |
| 5 | N-Acetyl-p-phenol | 70 | N-Acetyl-p-cyclohexanon (75,0 %) |

## Patentansprüche

1. Verfahren zur Herstellung von Cyclohexanonen der Formel 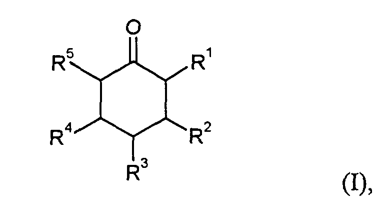 in der
R¹, R², R³, R⁴ und R⁵ unabhängig voneinander jeweils für Wasserstoff, Hydroxy, C₁-C₁₀-Alkyl, C₃-C₆-Cycloalkyl, C₆-C₁₀-Aryl, C₆-C₁₀-Aryl-CH₂-, C₆-C₁₀-Aryl-O-, C₁-C₁₀-Alkoxy, C₃-C₈-Cycloalkoxy, C₁-C₄-Alkylamino, Di-C₁-C₄-alkylamino, C₁-C₄-Acylamino, COOR⁶ mit R⁶ = Wasserstoff oder C₁-C₄-Alkyl oder R⁷-CH₂- mit R⁷ = Hydroxy, C₁-C₄-Alkoxy, C₁-C₄-Alkylamino oder Di-C₁-C₄-alkylamino stehen,
durch Hydrierung von Phenolen der Formel (II) 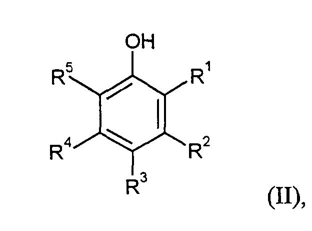 in der R¹ bis R⁵ die bei Formel (I) angegebene Bedeutung haben,
in Gegenwart eines Palladium-auf-Kohle-Katalysators bei 100 bis 250°C und 1 bis 20 bar Wasserstoffdruck, **dadurch gekennzeichnet, daß** man in Gegenwart von geradkettigen, verzweigten oder cyclischen Alkanen mit einem Siedepunkt bei Normaldruck von über 70°C als Lösungsmittel arbeitet.

2. Verfahren nach Anspruch 1, **dadurch gekennzeichnet, daß** in den Formeln (I) und (II) R¹, R², R³, R⁴ und R⁵ unabhängig voneinander für Wasserstoff, Hydroxy, C₁-C₆-Alkyl, C₁-C₆-Alkoxy oder C₁-C₄-Acylamino stehen und 1 bis 4 der Reste R¹ bis R⁵ von Wasserstoff verschieden sind.

3. Verfahren nach Ansprüchen 1 und 2, **dadurch gekennzeichnet, daß** in den Formeln (I) und (II) R¹, R², R⁴ und R⁵ für Wasserstoff und R³ für Hydroxy, C₁-C₆-Alkyl, C₁-C₆-Alkoxy oder C₁-C₄-Acylamino stehen.

4. Verfahren nach Ansprüchen 1 bis 3, **dadurch gekennzeichnet, daß** der Palladium-auf-Kohle-Katalysator 1 bis 15 Gew.-% Palladium enthält.

5. Verfahren nach Ansprüchen 1 bis 4, **dadurch gekennzeichnet**, das bezogen auf das Phenol der Formel (II) 0,001 bis 1 Gew.-% Katalysator (gerechnet als Pd-Metall) eingesetzt werden.

6. Verfahren nach Ansprüchen 1 bis 5, **dadurch gekennzeichnet, daß** man zusätzlich den Katalysator nicht als solchen oder in wasserfeuchter Form einsetzt, sondern ihn vorher mit einer Basenkomponente und 20 bis 200 Gew.-% Wasser, bezogen auf die Basenkomponente, vermischt.

7. Verfahren nach Ansprüchen 1 bis 6, **dadurch gekennzeichnet, daß** man als Lösungsmittel geradkettige oder verzweigte C₇-C₁₈-Alkane, unsubstituierte C₆-C₁₀-Cycloalkane und mit geradkettigen oder verzweigten C₁-C₁₀-Alkylgruppen substituierte C₆-C₁₀-Cycloalkane einsetzt.

8. Verfahren nach Ansprüchen 1 bis 7, **dadurch gekennzeichnet, daß** man als Lösungsmittel Isooctan, Cyclohexan oder Methylcyclohexan einsetzt.

9. Verfahren nach Ansprüchen 1 bis 8, **dadurch gekennzeichnet, daß** man auf 100 g des eingesetzten Phenols der Formel (II) als Lösungsmittel 10 bis 1 000 ml eines geradkettigen, verzweigten oder cyclischen Alkans mit einem Siedepunkt bei Normaldruck von über 70°C einsetzt.

10. Verfahren nach Ansprüchen 1 bis 6, **dadurch gekennzeichnet, daß** man als Basenkomponente Borax, Natriumacetat oder Dinatriumhydrogenphosphat, bezogen auf den Palladium-auf-Kohle-Katalysator in einer Menge von 5 bis 50 Gew.-% und, bezogen auf die Basenkomponente, 50 bis 150 Gew.-% Wasser einsetzt.

## Claims

1. Process for preparing cyclohexanones of the formula (I) 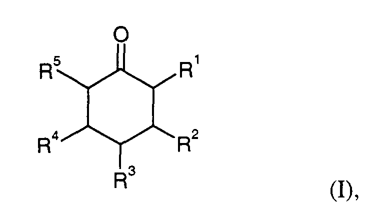 where
R¹, R², R³, R⁴ and R⁵ are each, independently of one another, hydrogen, hydroxy, C₁-C₁₀-alkyl, C₃-C₆-cycloalkyl, C₆-C₁₀-aryl, C₆-C₁₀-aryl-CH₂-, C₆-C₁₀-aryl-O-, C₁-C₁₀-alkoxy, C₃-C₈-cycloalkoxy, C₁-C₄-alkylamino, di-C₁-C₄-alkylamino, C₁-C₄-acylamino, COOR⁶ where R⁶ = hydrogen or C₁-C₄-alkyl or R⁷-CH₂- where R⁷ = hydroxy, C₁-C₄-alkoxy, C₁-C₄-alkylamino or di-C₁-C₄-alkylamino,
by hydrogenation of phenols of the formula (II) 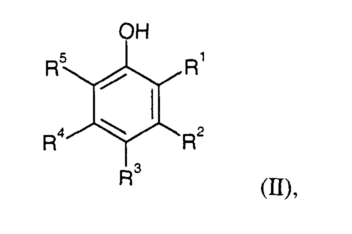 where R¹ to R⁵ are as defined for Formula (I),
in the presence of a palladium-on-carbon catalyst at from 100 to 250°C and from 1 to 20 bar of hydrogen pressure, **characterized in that** the reaction is carried out in the presence of straight-chain, branched or cyclic alkanes having a boiling point at atmospheric pressure of over 70°C as solvents.

2. Process according to Claim 1, **characterized in that**, in the formulae (I) and (II), R¹, R², R³, R⁴ and R⁵ represent, independently of one another, hydrogen, hydroxy, C₁-C₆-alkyl, C₁-C₆-alkoxy or C₁-C₄-acylamino and from 1 to 4 of the radicals R¹ to R⁵ are different from hydrogen.

3. Process according to Claims 1 and 2, **characterized in that**, in the formulae (I) and (II), R¹, R², R⁴ and R⁵ represent hydrogen and R³ represents hydroxy, C₁-C₆-alkyl, C₁-C₆-alkoxy or C₁-C₄-acylamino.

4. Process according to Claims 1 to 3, **characterized in that** the palladium-on-carbon catalyst contains from 1 to 15% by weight of palladium.

5. Process according to Claims 1 to 4, **characterized in that**, based on the phenol of the formula (II), from 0.001 to 1% by weight of catalyst (calculated as Pd metal) is used.

6. Process according to Claims 1 to 5, **characterized in that**, in addition, the catalyst is not used as such or in a form which is moist with water but is mixed beforehand with a base component and from 20 to 200% by weight of water, based on the base component.

7. Process according to Claims 1 to 6, **characterized in that** solvents used are straight-chain or branched C₇-C₁₈-alkanes, unsubstituted C₆-C₁₀-cycloalkanes and C₆-C₁₀-cycloalkanes substituted by straight-chain or branched C₁-C₁₀-alkyl groups.

8. Process according to Claims 1 to 7, **characterized in that** the solvent used is isooctane, cyclohexane or methylcyclohexane.

9. Process according to Claims 1 to 8, **characterized in that** from 10 to 1000 ml of a straight-chain, branched or cyclic alkane having a boiling point at atmospheric pressure of over 70°C is used as solvent per 100 g of the phenol of the formula (II) which is used.

10. Process according to Claims 1 to 6, **characterized in that** borax, sodium acetate or disodium hydrogen phosphate is used as base component in an amount of from 5 to 50% by weight, based on the palladium-on-carbon catalyst, and from 50 to 150% by weight of water, based on the base component, is used.

## Revendications

1. Procédé pour la préparation de cyclohexanones de formule 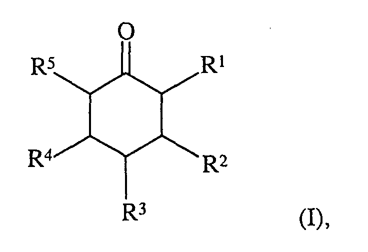 dans laquelle
R¹, R², R³, R⁴ et R⁵ représentent chacun, indépendamment les uns des autres, l'hydrogène, un groupe hydroxy, alkyle en C₁-C₁₀, cycloalkyle en C₃-C₆, aryle en C₆-C₁₀, (aryle en C₆-C₁₀)-CH₂-, (aryle en C₆-C₁₀)-O-, alcoxy en C₁-C₁₀, cycloalcoxy en C₃-C₈, alkylamino en C₁-C₄, di(alkyle en C₁-C₄)amino, acylamino en C₁-C₄, COOR⁶ avec R⁶ = H ou alkyle en C₁-C₄, ou R⁷-CH₂- avec R⁷ = hydroxy, alcoxy en C₁-C₄, alkylamino en C₁-C₄ ou di(alkyle en C₁-C₄)amino,
par hydrogénation de phénols de formule (II) 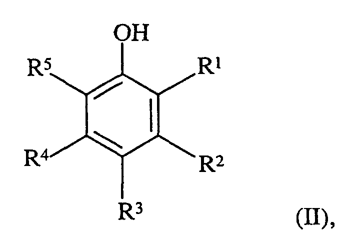 dans laquelle R¹ à R⁵ ont les significations indiquées en référence à la formule (I), en présence d'un catalyseur au palladium sur charbon à des températures de 100 à 250°C et des pressions d'hydrogène de 1 à 20 bars, **caractérisé en ce que** l'on opère en présence de solvants consistant en alcanes à chaîne droite, ramifiée ou cycliques dont le point d'ébullition à pression normale est supérieur à 70°C.

2. Procédé selon la revendication 1, **caractérisé en ce que**, dans les formules (I) et (II), R¹, R², R³, R⁴ et R⁵ représentent chacun, indépendamment les uns des autres, l'hydrogène, un groupe hydroxy, alkyle en C₁-C₆, alcoxy en C₁-C₆ ou acylamino ou C₁-C₄ et un à quatre des symboles R¹ à R⁵ ont une signification autre que l'hydrogène.

3. Procédé selon les revendications 1 et 2, **caractérisé en ce que**, dans les formules (I) et (II), R¹, R², R⁴ et R⁵ représentent l'hydrogène et R³ un groupe hydroxy, alkyle en C₁-C₆, alcoxy en C₁-C₆ ou acylamino en C₁-C₄.

4. Procédé selon les revendications 1 à 3, **caractérisé en ce que** le catalyseurs au palladium sur charbon contient 1 à 15 % en poids de palladium.

5. Procédé selon les revendications 1 à 4, **caractérisé en ce que**, par rapport au phénol de formule (II), on utilise de 0,001 à 1 % en poids du catalyseur (exprimé en Pd métallique).

6. Procédé selon les revendications 1 à 5, **caractérisé en outre en ce que** le catalyseur n'est pas mis en oeuvre tel quel ou à l'état humide, mais mélangé au préalable avec un composant basique et 20 à 200 % en poids d'eau par rapport au composant basique.

7. Procédé selon les revendications 1 à 6, **caractérisé en ce que** l'on utilise en tant que solvants des alcanes à chaîne droite ou ramifiée en C₇-C₁₈, des cycloalcanes non substitués en C₆-C₁₀ ou des cycloalcanes en C₁-C₁₀ substitués par des groupes alkyle à chaîne droite ou ramifiée en C₁-C₁₀.

8. Procédé selon les revendications 1 à 7, **caractérisé en ce que** l'on utilise en tant que solvant l'isooctane, le cyclohexane ou le méthylcyclohexane.

9. Procédé selon les revendications 1 à 8, **caractérisé en ce que**, pour 100 g du phénol de formule (II) mis en oeuvre, on utilise de 10 à 1000 ml d'un solvant consistant en un alcane à chaîne droite, ramifiée ou cyclique dont le point d'ébullition à pression normale est supérieur à 70°C.

10. Procédé selon les revendications 1 à 6, **caractérisé en ce que** l'on utilise en tant que composant basique le borax, l'acétate de sodium ou l'hydrogénophosphate disodique en quantité de 5 à 50 % du poids du catalyseur au palladium sur charbon et de l'eau en quantité de 50 à 150 % du poids du composant basique.
